# EUROPÄISCHE PATENTSCHRIFT

(11) **EP 1 499 257 B1**
(45) Veröffentlichungstag und Bekanntmachung des Hinweises auf die Patenterteilung: **08.09.2010**
(21) Anmeldenummer: 03720005.2
(22) Anmeldetag: 18.04.2003
(51) Int. Cl.: A61C 1/18, A61C 1/14, A61B 17/16

(54) **DREHMOMENTÜBERTRAGUNG FÜR EIN CHIRURGISCHES ODER DENTALES, ROTIERENDES WERKZEUG**
TORQUE TRANSMISSION FOR A SURGICAL OR DENTAL ROTATING TOOL
TRANSMISSION DE COUPLE POUR OUTIL ROTATIF CHIRURGICAL OU DENTAIRE

(30) Priorität: 18.04.2002 AT 6032002
(43) Veröffentlichungstag der Anmeldung: 26.01.2005
(60) Teilanmeldung: 10002986.7
(73) Patentinhaber: W & H Dentalwerk Bürmoos GmbH, 5111 Bürmoos (AT)
(72) Erfinder: HELFENBEIN, Gerald, A-5133 Gilgenberg (AT)
(74) Vertreter: Patentanwälte Barger, Piso & Partner
(86) Internationale Anmeldenummer: PCT/AT2003/000115
(87) Internationale Veröffentlichungsnummer: WO 2003/086222

(56) Entgegenhaltungen:
- EP-A- 0 470 324
- EP-A- 0 642 770
- WO-A-84/01099
- DE-A- 2 905 484
- DE-A- 3 040 537
- DE-A- 19 918 638
- US-A- 4 014 099

## Beschreibung

Die Erfindung betrifft ein Handstück für ein darin einsteckbares Werkzeug entsprechend dem Oberbegriff des Anspruches 1.

Ein derartiges Handstück und ein derartiges Werkzeug sind aus der DE 199 18 638 A bekannt. Dabei erfolgt die formschlüssige Übertragung des Drehmoments über eine abgeplattete Stelle des Schaftes des Werkzeuges, der eine entlang einer Sehne der Werkzeugaufnahme tief im Inneren des Handstückkopfes verlaufende Gegenfläche entspricht. Das Werkzeug weist eine axial durchgehende Bohrung auf und das Handstück eine Mündung, welche im montierten Zustand des Werkzeugs der axialen Bohrung gegenüberliegt. In diese kann eine Kanüle zum Zuführen von Kühlflüssigkeit eingehängt werden.

Ein nicht gattungsgemäßes Handstück ohne Zufuhrröhrchen, somit ohne Möglichkeit der Innenzufuhr von Kühlmitttel bzw. Spülmittel durch das Werkzeug und mit anderer Drehmomentübertragung ist aus der AT 375 011 B bekannt, bei dem, mit dem Werkzeug unverlierbar verbunden, ein Zahnrad, ein Lager für das Werkzeug und ein Teil des Kopfgehäuses als Einsatzteil vorgesehen ist. Der Grund für diese Maßnahmen liegt im Wunsch der möglichsten Miniaturisierung des Kopfes des Handstückes. Auf eine Werkzeugaufnahme wird aus diesem Grund verzichtet und dafür der hohe Aufwand mit dem Einsatzteil in Kauf genommen. Dass dieser, auf den restlichen Kopf (und das zweite Lager!) exakt abgestimmte Einsatzteil im Vergleich zum Werkzeug extrem teuer und wegen des Lagers extrem schlecht zu reinigen und zu desinfizieren ist, liegt auf der Hand.

Ein anderes, ebenfalls nicht gattungsgemäßes Handstück ohne Zufuhrröhrchen, mit einer Drehmomentübertragung über eine Art Klemmring ist aus der US 5,542,846 A bekannt. Dabei wird der Klemmring drehfest in einer Führungshülse gehalten und durch eine Feder schräg gestellt, wodurch er mittels Reibschluß den Schaft des Werkzeuges mitdreht

Es ist auf dem Gebiete der Chirurgie und der Dentalmedizin seit langem bekannt, Werkzeuge in Werkzeugaufnahmen von Handstücken einzusetzen und ihnen durch die Werkzeugaufnahme die beabsichtigte Dreh-, Schwing- oder Vibrationsbewegung zu vermitteln. Dazu gibt es eine Vielzahl unterschiedlicher Systeme und eine große Anzahl von speziell ausgebildeten und an die jeweiligen Werkzeugaufnahmen angepaßte Werkzeugschäfte. Von Bedeutung ist bei der Verwendung insbesondere im chirurgischen Bereich, bei dem in vielen Fällen größere Volumina von Knochen oder anderen harten Materialien abgetragen werden müssen als im Dentalbereich, dass Bohrer oder allgemein Werkzeuge verwendet werden, die in ihrem Inneren eine Zuleitung für ein Kühlmedium aufweisen. Dies dient einem wirksamen Kühlen der Arbeitsstelle und einem geordneten Austrag des abgespanten Materials von der Bearbeitungsstelle.

Darüberhinaus werden die gleichen Handstücke und damit die gleichen Werkzeugaufnahmen auch verwendet, um in gebohrte Löcher im Knochen Gewinde zu schneiden und um Implantate in die so geschaffenen Gewinde einzuschrauben. Insbesondere beim Einschrauben der Implantate werden Drehmomente übertragen, die deutlich höher liegen als beim Bohren des Loches im Knochen oder Zahn. Größenordnungsmäßig kann gesagt werden, dass hier Drehmomente bis 80 Newtonzentimeter angewandt werden, die nun über einen Werkzeugschaft mit einem üblichen Nominaldurchmesser von 2,35 mm übertragen werden müssen. Dabei ist noch zu bedenken, dass diese Werkzeugschäfte ja in ihrem Inneren ein Längsloch aufweisen, um das Zufuhrröhrchen der Werkzeugaufnahme, durch das die Kühlflüssigkeit geleitet wird, aufnehmen zu können, so dass nur ein dünner Mantelbereich des Werkzeugschaftes zur Übertragung des Drehmomentes zur Verfügung steht.

Die heute überwiegend verwendeten, dem eingangs genannte Stand der Technik entsprechenden, Werkzeugaufnahmen übertragen dabei das Drehmoment auf folgende Weise auf den Werkzeugschaft: Der Werkzeugschaft ist an seinem oberen Ende über eine Höhe von einigen Millimetern abgeplattet ausgebildet, diese Abplattung liegt an einem entsprechend vorstehenden, flachen, Teil der Werkzeugaufnahme an und bildet so eine formschlüssige Verbindung mit der Werkzeugaufnahme. In diesem Bereich des Werkzeugschaftes ist dessen Wandstärke extrem geschwächt, da sich, axial gesehen, knapp unterhalb dieser Abplattung eine Ringnut im Werkzeugschaft befindet, durch die die axiale Sicherung des Werkzeuges in der Werkzeugaufnahme bewerkstelligt wird.

Dadurch wird der zur Verfügung stehende Hebelarm, mit dem das Drehmoment übertragen wird, extrem klein und so kommt es beim Schneiden von Gewinden und insbesondere beim Eindrehen von Implantaten häufig dazu, dass der Werkzeugschaft in diesem Bereich plastisch deformiert wird und nicht mehr aus der Werkzeugaufnahme entfernt werden kann.

Ein anderer, sehr wesentlicher Nachteil des Standes der Technik ist, dass die Abdichtung zwischen dem koaxial zur Bohrerachse ins Innere des Langloches ragenden Zufuhrröhrchens der Werkzeugaufnahme gegenüber dem drehenden Bohrer nur extrem schwer zu bewerkstelligen ist. Die heute verwendeten Lösungen sehen so aus, dass dieses still stehende Zufuhrröhrchen weit aus dem Handstück vorsteht (und somit extrem gefährdet ist, was Beschädigungen betrifft), dass der Werkzeugschaft in einem Bereich, der nahe des unteren Endes des Zufuhrröhrchens liegt (etwa in der Mitte der Werkzeuglänge!!!), eine Verdickung aufweist, dass normal zur Werkzeugachse eine Bohrung durch diese Verdickung geschaffen wird, dass im Zuge der Herstellung des Werkzeuges ein Kern, der das Zufuhrröhrchen simuliert, in die Längsbohrung des Werkzeuges eingebracht wird, und dass sodann ein Dichtmaterial (Silikon) od.dgl. durch die Querbohrung eingespritzt wird und während des Erstarrens durch Bewegen des eingeschobenen Kernes daran gehindert wird, mit diesem zu verkleben.

Nach dem Erstarren bzw. Aushärten wird der Kern entnommen und die eingespritzte Dichtmasse bildet eine völlig irregulär gestaltete Dichtung auf etwa halber Länge des Werkzeuges.

Aus all dem Gesagten geht klar hervor, dass die Schaffung eines den Anforderungen bei der Benutzung problemlos genügenden Werkzeuges und seiner Aufnahme dringend notwendig ist und dass diese neue Werkzeugaufnahme bzw. das neue Werkzeug nicht nur den Erfordernissen während der Benutzung genügen soll, sondern dass auch die Herstellungskosten so herabgesetzt werden sollen, dass es möglich ist, eine weitere Forderung der Chirurgen und Zahnärzte zu erfüllen, dass nämlich derartige Werkzeuge nach einmaligem Gebrauch weggeworfen werden, was aus Gründen der Hygiene wünschenswert ist, aus Kostengründen derzeit aber noch nicht möglich ist. Man muß dabei bedenken, dass speziell die spanenden Teile des Werkzeuges Vertiefungen, Ausnehmungen, Hohlräume u.dgl. aufweisen, die bei invasiven Operationen, die mit ihnen durchgeführt werden, organisches Material des behandelten Patienten leicht aufnehmen und nur extrem schwer wieder abgeben.

Die Erfindung löst die gestellten Aufgaben durch die im kennzeichnenden Teil des Anspruchs 1 definierten Merkmale.

Auf diese Weise wird das Einsetzen erleichtert und bei Vermeiden jeder Schwächung des notgedrungen schon geringen Querschnittes des Werkzeuges in dem axialen Bereich, der vom Drehmoment belastet wird, das übertragbare Drehmoment bestmöglich erhöht.

Werkzeuge, die über keine Innenkühlung verfügen, beispielsweise die eingangs genannten Werkzeuge zum Einschrauben von Implantaten, können in dem Bereich, in dem das Drehmoment von der Werkzeugaufnahme übertragen wird, bereits voll ausgeführt sein und benötigen nur in ihrem oberen, praktisch drehrnomentfreien Bereich eine Ausnehmung, um das Zufuhrröhrchen aufnehmen zu können.

Erfindungsgemäß ist daher die Werkzeugaufnahme so ausgebildet, dass die Drehmomentübertragung in ihrem untersten, werkzeugseitigen Bereich formschlüssig erfolgt und dass das Zufuhrröhrchen für die Kühlflüssigkeit, axial gesehen, oberhalb der Drehmomentübertragung auf das Werkzeug endet.

In einer Ausgestaltung der Erfindung ist vorgesehen, dass das Element zur formschlüssigen Übertragung des Drehmomentes in der Werkzeugaufnahme einstückig mit dem Zahnrad ausgebildet ist, das das Drehmoment vom Antrieb überträgt. Auf diese Weise wird die beim Stand der Technik immer wieder vorkommende Verdrehung zwischen diesem Zahnrad und der eigentlichen Werkzeugaufnahme vermieden.

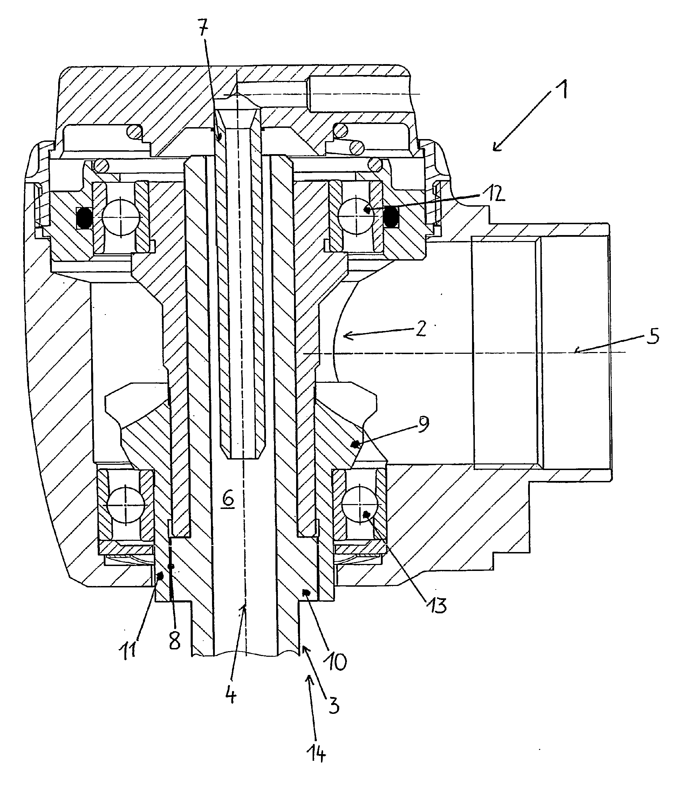

Die Erfindung wird im folgenden an Hand der Zeichnung, die aus nur einer Figur besteht, näher erläutert.

Die einzige Figur zeigt das vordere Ende eines erfindungsgemäßen Handstückes 1 im Schnitt entlang der Drehachse 4 des Werkzeuges 3 und der Drehachse 5 des (nicht dargestellten) Antriebes im Handstück 1.

Das Werkzeug 3, im dargestellten Fall ein Bohrer, Fräser od.dgl. mit Innenkühlung weist zur Zuleitung der Kühlflüssigkeit eine Längsbohrung 6 auf, die vom oberen Schaftende bis zu dem, nicht dargestellten, eigentlichen Arbeitsbereich führt, wo durch entsprechende Kanäle die Durchleitung der Kühlflüssigkeit durch den Werkzeugkopf zur eigentlichen Bearbeitungsstelle erfolgt. Um die Kühlflüssigkeit in die Längsbohrung 6 des Werkzeuges 3 einzubringen, ist in der Werkzeugaufnahme 2 ein Zufuhrröhrchen 7 vorgesehen, das koaxial mit dem Werkzeug 3 verläuft und dessen Abmessungen so gewählt sind, dass sein Außendurchmesser kleiner ist als der Innendurchmesser der Längsbohrung 6 und dass es, axial gesehen, in einem Bereich der Werkzeugaufhahme 2 endet, der noch oberhalb des Bereiches 8 der Drehmomentübertragung liegt.

Dieser Bereich 8 der Drehmomentübertragung liegt im untersten Abschnitt des Handstückkopfes. Mit "unten" bzw. "oben" werden in der Anmeldung und den Ansprüchen die Richtungen verstanden, wie sie beim Betrachten der Zeichnung vorliegen. In der Werkzeugaufnahme ist "unten" die Seite, von der das Werkzeug eingesteckt wird, für das Werkzeug ist "unten" die Seite, an der sich der Werkzeugkopf befindet und "oben" die Seite des Werkzeugschaftes, in die das Zufuhrröhrchen 7 gesteckt wird.

Die Drehmomentübertragung erfolgt einerseits mittels eines hülsenartigen Fortsatzes 11 des Antriebszahnrades 9, der sich bis in diesen Bereich 8 erstreckt, andererseits weist der Schaft des Werkzeuges 3 in diesem Bereich 8 eine Verdickung 10 auf, dies jeweils mit der Maßgabe, dass die Innenkontur des hülsenartigen Fortsatzes 11 mit der Außenkontur der Verdickung 10 korrespondiert und dass durch diese Übereinstimmung eine formschlüssige Verdrehsicherung geschaffen wird.

Wie aus dem Gesagten leicht entnehmbar ist und im Zusammenhang mit der Zeichnung direkt ersichtlich ist, wird das Drehmoment vom Zahnrad 9 über seinen hülsenartigen Fortsatz 11 und die formschlüssige Verbindung mit der Verdickung 10 auf den Schaft des Werkzeuges 3 übertragen, der von diesem axialen Bereich an nach unten zum (nicht dargestellten) Bearbeitungskopf hin, keinerlei Schwächung erleidet.

Wenn statt des dargestellten Werkzeuges 3 mit Innenkühlung ein Schlüssel oder Übertragungsstift od.dgl. eingespannt wird, mit dem beispielsweise ein Implantat eingeschraubt wird, so kann dieser, wie leicht ersichtlich, unmittelbar unterhalb des Bereiches, in dem das Zufuhrröhrchen 7 endet, voll ausgebildet sein, d.h., dass für den gesamten Bereich, der mit dem hohen Drehmoment für das Eindrehen beaufschlagt wird, der volle Querschnitt zur Verfügung steht. Dieser Querschnitt kann u.U. auch größer sein als der des dargestellten Werkzeuges 3, da, wie aus der Figur ersichtlich ist, vom Bereich der Drehmomentübertragung 8 an, der Werkzeugkopf 1 und die Werkzeugaufnahme 2 es erlauben, dass ein Werkzeugschaft mit größerem Durchmesser (in diesem Bereich, natürlich nicht im oberen Bereich der Werkzeugaufnahme) verwendet wird.

Wenn man auf die Verwendung von Bohrern mit dem oben genannten Standarddurchmesser von 2,35 mm verzichtet und beispielsweise auf Durchmesser von 3 mm übergeht, so kann man die Längsbohrung für die Zufuhr der Kühlflüssigkeit durch spanende Bearbeitung schaffen und ist nicht mehr auf die extrem teure Methode des Senk-Erodierens angewiesen. Die Zahl der Einsatzmöglichkeiten für derartige Bohrer nimmt bei der dadurch erzielbaren Kostenreduktion stark zu.

Die Details der Ausführung, wie die verwendbaren Materialien, die Herstellungsverfahren, die Anpassung an bestehende Systeme sind für den Fachmann in Kenntnis der Erfindung, leicht bestimmbar und brauchen hier nicht abgehandelt zu werden.

## Patentansprüche

1. Handstück für ein chirurgisches oder dentales, in den Kopf des Handstückes (1) durch eine Einstecköffnung (14) in eine Werkzeugaufnahme (2) einsteckbares, mittels zweier, axialen Abstand voneinander aufweisenden Lager (12,13) rotierend gelagertes Werkzeug (3) mit einem Bereich (8) zur formschlüssigen Drehmomentübertragung auf das Werkzeug (3), wobei des Bereich (8) zur formschlüssigen Drehmomentübertragung im Bereich des Lagers (13) vorgesehen ist, das der Einstecköffnung (14) benachbart ist, wobei das Handstück ein feststehendes Zufuhrröhrchen (7) für Kühlflüssigkeit aufweist, welches axial in die Werkzeugaufnahme (2) ragt und welches axial gesehen oberhalb des Bereichs (8) zur formschlüssigen Drehmomentübertragung endet, **dadurch gekennzeichnet, dass** der Bereich (8) zur formschlüssigen Drehmomentübertragung durch eine polygonale Ausnehmung der Einstecköffnung (14) der Werkzeugaufnahme (2) gebildet ist.

2. Handstück nach Anspruch 1, **dadurch gekennzeichnet, dass** der Bereich (8) zur formschlüssigen Drehmomentübertragung außerhalb der beiden Lager angeordnet ist.

3. Handstück nach Anspruch 1 oder 2, **dadurch gekennzeichnet, dass** das Zufuhrröhrchen (7) im axialen Bereich zwischen den beiden Lagern (12, 13) endet.

4. Handstück nach einem des voranstehendem Ansprüche, **dadurch gekennzeichnet, dass** der Bereich (8) zur formschlüssigen Drehmomentübertragung an der Innenseite eines hülsenartigen Fortsatzes (11) vorgesehen ist.

5. Handstück nach Anspruch 4, **dadurch gekennzeichnet, dass** der hülsenartige Forbatz (11) mit einem Zahnrad (9) verbunden ist.

6. Handstück nach Anspruch 5, **dadurch gekennzeichnet, dass** der hülsenartige Fortsatz (11) einstückig mit dem Zahnrad (9) ausgebildet ist.

7. Handstück nach einem der voranstehenden Ansprüche , **dadurch gekennzeichnet, dass** die polygonale Ausnehmung ein regelmäßiges Sechseck ist.

8. Handstück mit einem Werkzeug, **gekennzeichnet durch** ein Handstück nach einem der voranstehenden Ausprüche und ein Werkzeug mit einem Schaft (3), der im Abstand vom handstückseitigen Ende des Werkzeuges einen Bereich (10) zur formschlüssigen Drehmomentübertragung aufweist, wobei der Bereich (10) einen polygonalen Querschnitt, insbesondere einen polygonalen Querschnitt in Form eines regelmäßigen Sechseckes, aufweist.

9. Handstück nach Anspruch 8, **dadurch gekennzeichnet, dass** das Werkzeug an oder nahe an seinem handstückseitigen Ende eine radial nach innen ragende Dichtung aufweist.

10. Handstück nach einem der Ansprüche 8 oder 9, **dadurch gekennzeichnet, dass** das Werkzeug in seinem axialen Bereich zwischen dem Bereich (10) zur formschlüssigen Drehmomentübertragung und seinem handstückfernen Ende einen geschlossenen Querschnitt aufweist.

11. Handstück nach einem der Ansprüche 8 oder 9, **dadurch gekennzeichnet, dass** der Werkzeugschaft (3) eine Längsbohrung (6) zum Transport von Kühlflüssigkeit zur Arbeitsstelle aufweist.

## Claims

1. Handpiece for a surgical or a dental rotary mounted tool (3), which can be inserted in the head of the handpiece (1) through an insertion hole (14) into a tool holder (2) and is rotationally supported by means of two axially separated bearings (12, 13), with an area (8) for positive torque transmission to the tool (3), whereby the area (8) for positive torque transmission is provided in the area of the bearing (13), which is adjacent the insertion hole (14), whereby the hand piece is provided with a fixed feed tube (7) for cooling liquid, which extends axial into the tool holder (2) and which ends axially above the area (8) for positive torque transmission, **characterised in that** the area (8) for positive torque transmission is composed of a polygonal recess of the insertion hole of the tool holder (2).

2. Handpiece in accordance with claim 1, **characterised in that** the area (8) for positive torque transmission is arranged beyond both bearings.

3. Handpiece in accordance with claim 1 or 2, **characterised in that** the feed tube (7) ends in the axial region between the two bearings (12, 13).

4. Handpiece in accordance with any of the preceding claims, **characterised in that** the area (8) for positive torque transmission is provided on the inner surface of a sleeve-like extension (11).

5. Handpiece in accordance with claim 4, **characterised in that** the sleeve-like extension (11) is connected with a gear wheel (9).

6. Handpiece in accordance with claim 5, **characterised in that** the sleeve-like extension (11) is integrally formed with the gear wheel (9).

7. Handpiece in accordance with any of the preceding claims, **characterised in that** polygonal recess is a regular hexagon.

8. Handpiece with a tool, **characterised by** a handpiece in accordance with any of the preceding claims and a tool with a shaft (3), which has a region (10) for positive torque transmission in a distance of the lateral handpiece end, whereby the region (10) has a polygonal cross-section, especially a polygonal cross-section with the form of a regular hexagon.

9. Handpiece in accordance with claim 8, **characterised in that** the tool has at or near its lateral handpiece end, a seal that extends radially inwardly.

10. Handpiece in accordance with claim 8 or 9, **characterised in that** the tool has a closed cross-section in its axial region between the region (10) for positive torque transmission and its end remote from the handpiece.

11. Handpiece in accordance with claim 8 or 9, **characterised in that** the tool shaft (3) has a longitudinal drill (6) for the transport of cooling liquid to the working position.

## Revendications

1. Poignée porte-outil pour un outil (3) chirurgical ou dentaire, qui peut être inséré dans un logement de réception d'outil (2) dans la tête de la poignée porte-outil (1) par une ouverture d'insertion (14), et est monté rotatif au moyen de deux paliers ou roulements (12, 13) présentant une distance d'espacement axiale l'un de l'autre, une zone (8) étant prévue pour une transmission mécanique positive de couple à l'outil (3), la zone (8) pour la transmission mécanique positive de couple étant prévue dans zone du palier ou roulement (13) qui est voisin de l'ouverture d'insertion (14), et la poignée porte-outil comportant un petit tube d'amenée (7) fixe, pour du liquide de refroidissement, qui s'engage axialement dans le logement de réception d'outil (2) et se termine, vu axialement, au-dessus de la zone (8) pour la transmission mécanique positive de couple,
**caractérisée en ce que** la zone (8) pour la transmission mécanique positive de couple est formée par un évidement polygonal de l'ouverture d'insertion (14) du logement de réception d'outil (2).

2. Poignée porte-outil selon la revendication 1, **caractérisée en ce que** la zone (8) pour la transmission mécanique positive de couple est agencée à l'extérieur des deux paliers ou roulements.

3. Poignée porte-outil selon la revendication 1 ou la revendication 2, **caractérisée en ce que** le petit tube d'amenée (7) se termine dans la zone axiale entre les deux paliers ou roulements (12, 13).

4. Poignée porte-outil selon l'une des revendications précédentes, **caractérisée en ce que** la zone (8) pour la transmission mécanique positive de couple est prévue sur le côté intérieur d'un prolongement (11) en forme de manchon.

5. Poignée porte-outil selon la revendication 4, **caractérisée en ce que** le prolongement (11) en forme de manchon est relié à une roue dentée (9).

6. Poignée porte-outil selon la revendication 5, **caractérisée en ce que** le prolongement (11) en forme de manchon est réalisé d'un seul tenant avec la roue dentée (9).

7. Poignée porte-outil selon l'une des revendications précédentes, **caractérisée en ce que** l'évidement polygonal est un hexagone régulier.

8. Poignée porte-outil avec un outil, **caractérisée par** une poignée porte-outil selon l'une des revendications précédentes, et par un outil comportant une queue d'outil (3), qui présente, à distance de l'extrémité côté poignée porte-outil de l'outil, une zone (10) pour la transmission mécanique positive de couple, ladite zone (10) présentant une section transversale polygonale, notamment une section transversale polygonale sous la forme d'un hexagone régulier.

9. Poignée porte-outil selon la revendication 8, **caractérisée en ce que** l'outil présente au niveau de son extrémité côté poignée porte-outil ou à proximité de celle-ci, un joint d'étanchéité faisant saillie radialement vers l'intérieur.

10. Poignée porte-outil selon l'une des revendications 8 ou 9, **caractérisée en ce que** l'outil présente une section transversale fermée dans sa zone axiale entre ladite zone (10) pour la transmission mécanique positive de couple et son extrémité éloignée de la poignée porte-outil.

11. Poignée porte-outil selon l'une des revendications 8 ou 9, **caractérisée en ce que** la queue d'outil (3) présente un alésage longitudinal (6) pour le transport de liquide de refroidissement à la zone de travail.
